# EUROPÄISCHE PATENTSCHRIFT

(11) **EP 0 847 392 B1**
(45) Veröffentlichungstag und Bekanntmachung des Hinweises auf die Patenterteilung: **08.12.1999**
(21) Anmeldenummer: 96928474.4
(22) Anmeldetag: 19.08.1996
(51) Int. Cl.: C07D 239/96

(54) **VERFAHREN ZUR HERSTELLUNG VON CHINAZOLINDERIVATEN**
PROCESS FOR PRODUCING QUINAZOLINE DERIVATIVES
PROCEDE DE PRODUCTION DE DERIVES DE QUINAZOLINE

(30) Priorität: 31.08.1995 DE 19532052
(43) Veröffentlichungstag der Anmeldung: 17.06.1998
(73) Patentinhaber: Clariant GmbH, 65929 Frankfurt am Main (DE)
(72) Erfinder: PAPENFUHS, Theodor, D-60433 Frankfurt am Main (DE); PFIRMANN, Ralf, D-64347 Griesheim (DE); KRAUSE, Stefan, D-65929 Frankfurt (DE); NEUMANN-GRIMM, Doris, D-60388 Frankfurt am Main (DE)
(86) Internationale Anmeldenummer: EP9603632
(87) Internationale Veröffentlichungsnummer: WO9708154

(56) Entgegenhaltungen:
- EP-A- 0 176 333
- MONATSHEFTE FUR CHEMIE, Bd. 118, 1987, WIEN AT, Seiten 71-79, XP002016462 M. SUSSE,S. JOHNE: "CHINAZOLINCARBONSAUREN,10.MITT. (I)." in der Anmeldung erwähnt

## Beschreibung

Die vorliegende Erfindung betrifft ein neues Verfahren zur Herstellung von 1,2,3,4-Tetrahydro-2,4-dioxochinazolin-1-ylessigsäurederivaten.

1,2,3,4-Tetrahydro-2,4-dioxochinazolin-1-ylessigsäure und deren Derivate sind wichtige Zwischenprodukte für die Herstellung von Aldose-Reduktase-Hemmern (EP 218 999).

In J. Am. Chem. Soc. (1933), S. 2113-2116 wird die Umsetzung von N-Ethylanthranilsäure mit Natriumcyanat und Essigsäure und nachfolgender Zugabe von Natriumhydroxid zu 1,2,3,4-Tetrahydro-1-ethyl-2,4-dioxochinazolin beschrieben. Nachteilig an diesem Verfahren ist jedoch die geringe Raumausbeute aufgrund der verdünnten Reaktionslösung und der sehr große Natriumhydroxidüberschuß.
In Monatsh. Chem. (1987) 118; S. 71-79 wird die Umsetzung von N-(Methoxycarbonylmethyl)anthranilsäuremethylester mit Kaliumcyanat in Eisessig zu 1,2,3,4-Tetrahydro-2,4-dioxochinazolin-1-ylessigsäuremethylester beschrieben. Obwohl 10 Äquivalente Kaliumcyanat eingesetzt werden, beträgt die Ausbeute jedoch nur 19 %.

Es bestand daher der Bedarf nach einem effizienten Verfahren zur Umsetzung von Anthranilsäuren zu 1,2,3,4-Tetrahydro-2,4-dioxochinazolin-1-ylessigsäurederivaten.

Diese Aufgabe wird gelöst durch ein Verfahren zur Herstellung von 1,2,3,4-Tetrahydro-2,4-dioxo-chinazolin-1-ylessigsäurederivaten der Formel (I) 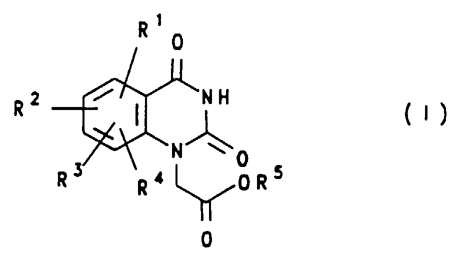 worin
- R¹, R², R³, R⁴: unabhängig voneinander Wasserstoff, Halogen, NO₂, (C₁-C₆)Alkoxy, (C₁-C₆)Alkyl, halogensubstituiertes (C₁-C₆)Alkyl
- R⁵: Wasserstoff, (C₁-C₆)Alkyl, Phenyl, wobei der Alkyl- oder Phenylrest auch mit Halogenatomen substituiert sein kann, bedeuten,
dadurch gekennzeichnet, daß man ein Anthranilsäurederivat der Formel (II) 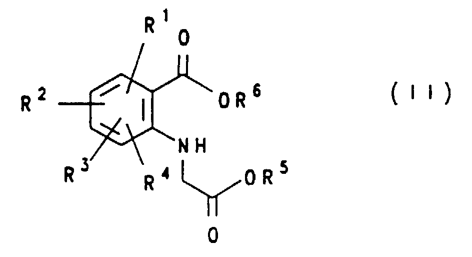 worin
R¹ bis R⁵ die oben erwähnte Bedeutung besitzen und R⁶ für Wasserstoff, (C₁-C₆)Alkyl oder Phenyl steht, wobei der Alkyl- oder Phenylrest auch mit Halogenatomen substituiert sein kann, mit einem Metallcyanat und Chlorwasserstoff in Gegenwart eines inerten Lösungsmittels umsetzt.

Wichtig ist das Verfahren zur Umsetzung von Verbindungen der Formel (II) worin R¹, R², R³, R⁴ Wasserstoff, Fluor, Chlor, (C₁-C₄)Alkoxy, (C₁-C₄)Alkyl, chlor- oder fluorsubstituiertes (C₁-C₄)Alkyl und R⁵, R⁶ Wasserstoff, (C₁-C₄)Alkyl oder Phenyl bedeuten.
Hier sind die Umsetzungen von Verbindungen der Formel (II) von Bedeutung worin R¹, R², R³, R⁴ Wasserstoff, Fluor, Chlor, Methyl oder Ethyl und R⁵, R⁶ Wasserstoff, Methyl oder Ethyl bedeuten.
Insbesondere wichtig ist das Verfahren auch zur Herstellung von Verbindungen der Formel (I) worin zwei, bevorzugt drei der Reste R¹, R², R³, R⁴ für Wasserstoff stehen.
Von besonderem Interesse ist das Verfahren zur Herstellung von 1,2,3,4-Tetrahydro-7-chloro-2,4-dioxochinazolin-1-ylessigsäure und deren Methyl- und Ethylester.

Es hat sich in vielen Fällen bewährt, zur Reaktion die Anthranilsäurederivate der Formel (II) in einem Lösungsmittel vorzulegen.
Die Anthranilsäure kann dabei in gelöster Form oder als Suspension vorliegen. Als Lösungsmittel können aprotische Lösungsmittel oder protisch organische Lösungsmittel oder Mischungen dieser Lösungsmittel verwendet werden. Vorteilhaft ist der Einsatz von polaren aprotischen Lösungsmittel, die unter den Reaktionsbedingungen keine Reaktion zeigen, z.B. Sulfolan, Dimethylsulfoxid, Dimethylsulfon, Diphenylsulfon, Tetramethylharnstoff oder Mischungen derselben.
Die Konzentration an Anthranilsäure im Lösungsmittel liegt zwischen 1 und 50 Gew.-%, vorteilhaft zwischen 1,5 und 20 Gew.-%, bevorzugt zwischen 3 und 10 Gew.-%.
Als Metallcyanate können Alkali- und Erdalkalicyanate eingesetzt werden, auch Mischungen derselben. Vorteilhaft ist die Verwendung von Natrium- oder Kaliumcyanat oder Mischungen derselben.
Es hat sich als günstig erwiesen die Metallcyanate in Mengen zwischen 0.8 und 20 Äquivalenten, insbesondere zwischen 2 und 5 Äquivalenten bezogen auf die Anthranilsäurederivate zuzugeben. Die Metallcyanate können zusammen mit der Anthranilsäure vorgelegt oder kontinuierlich oder in Portionen zugegeben weden. Chlorwasserstoff kann gasförmig oder als nichtwäßrige Lösung in einer Portion, in mehreren Portionen oder kontinuierlich zugeführt werden, vorteilhaft ist das kontinuierliche Einleiten von Chlorwasserstoff bis zum Reaktionsende. Die Reaktionspartner Metallcyanat, Chlorwasserstoff und Anthranilsäure können zur Reaktion in beliebiger Reihenfolge zugegeben werden, vorteilhaft ist das Vorlegen von Anthranilsäure und Metallcyanat und das anschließende Dosieren von Chlorwasserstoff oder das Vorlegen von Chlorwasserstoff und Anthranilsäure und das anschließende Dosieren von Metallcyanat oder das Vorlegen von Kaliumcyanat und Chlorwasserstoff und das Dosieren von Anthranilsäure oder Kombination derselben.
Die Reaktionstemperatur liegt zwischen dem Festpunkt des Lösungsmittels und 150°C, vorteilhaft zwischen 0 und 100°C, besonders vorteilhaft zwischen 20 und 75°C.
Die Reaktionszeiten betragen zwischen 15 min und 24 h, vorteilhaft zwischen 1 und 15 h, besonders vorteilhaft zwischen 2 und 10 h.
Die Reaktion kann bei Unter-, Über- oder Normaldruck durchgeführt werden, vorteilhaft ist die Durchführung bei Normaldruck.

Daß der glatte Reaktionsverlauf besonders überraschend ist, wird durch das Vergleichsbeispiel belegt. Es zeigt, daß die Reaktion keineswegs generell durch Säuren katalysiert werden kann, sondern daß die Wahl der Säure HCI von entscheidender Bedeutung ist. Die Synthese der Anthranilsäuren mit der allgemeinen Formel (II) ist in der deutschen Patentanmeldung Aktenzeichen 195 32 054.9 und DRP 11911 beschrieben.

Die folgenden Beispiele erläutern die Erfindung, ohne sie zu beschränken.

### Beispiel 1:

### Herstellung von 1,2,3,4-Tetrahydro-7-chloro-2,4-dioxochinazolin-1-ylessigsäureethylester aus 4-Chlor-N-(ethoxycarbonylmethyl)anthranilsäure.

Zu 1 g 4-Chlor-N-(ethoxycarbonylmethyl)anthranilsäure und 1.6 g Kaliumcyanat in 20 ml Sulfolan wird bei 50°C Chlorwasserstoff eingeleitet, bis eine HPLC-Analyse vollständige Umsetzung anzeigt. Das Produkt wird durch Zugabe von Wasser ausgefällt, abfiltriert, mit Wasser gewaschen und getrocknet. Man erhält 0,95 g (87 %) 1,2,3,4-Tetrahydro-7-chloro-2,4-dioxochinazolin-1-ylessigsäureethylester.
Smp: 242-243°C
¹H NMR (DMF): 1.25 (t, J = 7.0 Hz, -CH₃), 4.21 (q, J = 7.0 Hz, O-CH₂-), 5.00 (s, N-CH₂), 7.38 (dd, J = 1.8 Hz, J = 8.2 Hz, 6-H), 7.70 (d, J = 1.8 Hz, 8-H), 8.09 (d, J = 8.2 Hz, 5-H), 11.86 (s, N-H).

### Beispiel 2:

### Herstellung von 1,2,3,4-Tetrahydro-7-chloro-2,4-dioxochinazolin-1-ylessigsäuremethylester aus 4-Chlor-N-(methoxycarbonylmethyl)-anthranilsäuremethylester

Zu 1 g 4-Chlor-N-(methoxycarbonylmethyl)anthranilsäuremethylester und 1.6 g Kaliumcyanat in 20 ml Sulfolan wird bei 50°C Chlorwasserstoff eingeleitet, bis eine HPLC Analyse vollständige Umsetzung anzeigt. Das Produkt wird durch Zugabe von Wasser ausgefällt, abfiltriert, mit Wasser gewaschen und getrocknet. Man erhält 0.59 g (57 %) 1,2,3,4-Tetrahydro-7-chloro-2,4-dioxochinazolin-1-ylessigsäuremethylester.
Smp.: 255-258°C
¹H-NMR (DMSO-d₆): 3.71 (s, -CH₃), 4.92 (s, N-CH₂), 7.33 (dd, J = 1.5 Hz, J = 8.5 Hz, 6-H), 7.60 (d, J = 1.5 Hz, 8-H), 8.01 (d, J = 8.5 Hz, 5-H).

### Beispiel 3

### Herstellung von 1,2,3,4-Tetrahydro-7-chloro-2,4-dioxochinazolin-1-ylessigsäure aus N-Carboxymethylen-4-chloranthranilsäure

In eine Mischung aus 20 ml Sulfon und 1.6 g Kaliumcyanat wird bei 25°C bis zur Sättigung Chlorwasserstoff eingeleitet. Anschließend werden 1 g N-Carboxymethylen-4-chloranthranilsäure zugegeben und auf 50°C erwärmt. Nach drei Stunden zeigt eine HPLC-Analyse vollständige Umsetzung zu 1,2,3,4-Tetrahydro-7-chloro-2,4-dioxo-chinazolin-1-ylessigsäure an. Smp: 278 - 282°C
¹H-NMR:
(DMSO-d₆): 4.71 (s, N-CH₂), 7.30 (dd, J = 1.5 Hz, J = 8.5 Hz, 6H), 7.41 (d, J = 1.5 Hz, 8-H), 7.99 (d, J = 8.5 Hz, 5-H), 11.75 (s, N-H).

### Beispiel 4

### Herstellung von 1,2,3,4-Tetrahydro-7-chlor-2,4-dioxochinazolin-1-ylessigsäureethylester aus 4-Chlor-N-(ethoxycarbonylmethyl)anthranilsäure

Zu 700 g Sulfolan werden bei 15 - 20°C über fünf Stunden insgesamt 128 g Kaliumcyanat und 144 g Chlorwasserstoff dosiert. Dazu werden zunächst 14,4 g Chlorwasserstoff eingeleitet und anschließend 12,8 g Kaliumcyanat zugegeben; dieser Vorgang wird 10 mal wiederholt, bis die gesamte Menge an Chlorwasserstoff und Kaliumcyanat zugegeben wurde. Anschließend werden bei 45 - 50°C 117 g 4-Chlor-N-(ethoxycarbonylmethyl)anthranilsäure in 2 Stunden zugegeben und 15 min nachgerührt. Die Suspension wird abfiltriert und mit Wasser sulfolan- und salzfrei gewaschen. Nach dem Trocknen werden 121 g (95 %) 1,2,3,4-Tetrahydro-7-chlor-2,4-dioxochinazolin-1-ylessigsäureethylester erhalten.

### Beispiel 5

### Herstellung von 1,2,3,4-Tetrahydro-7-chlor-2,4-dioxochinazolin-1-ylessigsäure-iso-propyl-ester aus 7-Chlor-N-(iso-propoxycarbonylmethyl)anthranilsäure

In eine Mischung aus 10 g Sulfolan und 1,3 g Kaliumcyanat wird bei 20°C bis zur Sättigung Chlorwasserstoff eingeleitet. Anschließend wird bei 50°C 1 g 7-Chlor-N-(iso-propoxy-carbonylmethyl)anthranilsäure zugegeben und 30 Minuten nachgerührt. Das Reaktionsgemisch wird auf Wasser gegeben, abfiltriert mit Wasser gewaschen und getrocknet. Es werden 0,85 g (78 %) 1,2,3,4-Tetrahydro-7-chlor-2,4-dioxochinazolin-1-ylessigsäure-iso-propyl-ester erhalten.

¹H-NMR (DMSO-d₆): 1.21 (d, J = 6.3 Hz, H₃C-C-CH₃), 4,88 (s, N-CH₂), 4.97 (sept, J = 6.3 Hz, O-CH), 7.33 (dd, J = 1.7 Hz, J = 8.4 Hz, 6-H), 7.53 (d, J = 1.7 Hz, 8-H), 8.00 (d, J = 8.4 Hz, 5-H), 11.83 (s, N-H).

### Beispiel 6

### Herstellung von 1,2,3,4-Tetrahydro-7-chlor-2,4-dioxochinazolin-1-ylessigsäure-n-hexyl-ester aus 7-Chlor-N-(n-hexoxycarbonylmethyl)anthranilsäure

Durchführung wie unter Beispiel 5
Ausbeute: 0.95 g (88 %)

¹H-NMR (DMSO-d₆): 0.84 (m, n-Hexyl), 1.22 (m, n-Hexyl), 1.55 (m, n-Hexyl) 4.07 (m, n-Hexyl), 4.92 (s, N-CH₂), 7.33 (dd, J = 1.5 Hz, J = 8.4 Hz, 6-H), 7.55 (d, J = 1.5 Hz, 8-H), 8.01 (d, J = 8.4 Hz, 5-H), 11.84 (s, N-H).

### Beispiel 7

### Herstellung von 1,2,3,4-Tetrahydro-7-chlor-2,4-dioxochinazolin-1-ylessigsäurebenzylester aus 7-Chlor-N-(benzoxycarbonylmethyl)anthranilsäure

Durchführung wie unter Beispiel 5.
Ausbeute: 0.80 g (74 %)
¹H-NMR (DMSO-d₆): 5.01 (s, -CH₂-), 5.22 (s, -CH₂-), 7.36 (m, 6-H, aromat.-H), 7.59 (d, J = 1.7 Hz, 8-H), 8.01 (d, J = 8.4 Hz, 5-H), 11.86 (s, N-H).

### Beispiel 8

### Herstellung von 1,2,3,4-Tetrahydro-5-fluor-2,4-dioxochinazolin-1-ylessigsäureethylester aus 6-Fluor-N-(ethoxycarbonylmethyl)anthranilsäure

Durchführung wie unter Beispiel 5.
Ausbeute: 0.60 g (54 %).
¹H-NMR (DMSO-d₆): 1.21 (t, J = 7.1 Hz, -CH₃), 4.16 (q, J = 7.1 Hz, -O-CH₂), 4.89 (s, N-CH₂), 7.06 (dd, J = 8.5 Hz, J = 11.0 Hz, aromat.-H), 7.14 (d, J = 18.6 Hz, aromat.-H), 7.70 (dd, J = 5.8 Hz, J = 8.5 HZ, aromat.-H), 11.71 (s, N-H).

### Beispiel 9

Herstellung von 1,2,3,4-Tetrahydro-8-chlor-2,4-dioxochinazolin-1-ylessigsäureethylester aus 3-Chlor-N-(ethoxycarbonylmethyl)anthranilsäure
Durchführung wie unter Beispiel 5.
Ausbeute: 0.70 g (64 %).
¹H-NMR (DMSO-d₆): 1.21 (t, J = 7.1 Hz, -CH₃), 4.19 (q, J = 7.1 Hz, -O-CH₂), 5.04 (s, N-CH₂), 7.30 (dd, J₁ = J₂ = 7.8 Hz, aromat.-H), 7.81 (dd, J = 1.7 Hz, J = 7.8 Hz, aromat.-H), 8.04 (dd, J = 1.7 Hz, J = 7.8 Hz, aromat.-H), 11.97 (s, N-H).

### Beispiel 10

### Herstellung von 1,2,3,4-Tetrahydro-6-nitro-2,4-dioxochinazolin-1-ylessigsäureethylester aus 5-Nitro-N-(ethoxycarbonylmethyl)anthranilsäure

Durchführung wie unter Beispiel 5.
¹H-NMR (DMSO-d₆): 1.22 (t, J = 1.22 Hz, -CH₃), 4.18 (q, J = 7.1 Hz, O-CH₂), 4.98 (s, N-CH₂), 12.19 (s, N-H).

### Vergleichsbeispiel 1

Zu 1 g 4-Chlor-N-(ethoxycarbonylmethyl)anthranilsäure und 1.6 g Kaliumcyanat in 20 ml Sulfolan werden bei 50°C 1 ml konzentrierte Schwefelsäure zugetropft. Nach 5 h zeigt eine HPLC-Untersuchung keine Umsetzung an.

### Vergleichsbeispiel 2

1 g 4-Chlor-N-(ethoxycarbonylmethyl)anthranilsäure und 1.6 g Kaliulcyanat werden in 20 ml Sulfolan mit 2.4 g Essigsäure versetzt und bei 50°C gerührt. Nach 6 h Reaktionszeit zeigt eine HPLC-Untersuchung ca. 5 % Umsatz an.

## Patentansprüche

1. Verfahren zur Herstellung von 1,2,3,4-Tetrahydro-2,4-dioxo-chinazolin-1-ylessigsäure-Derivaten der Formel (I) 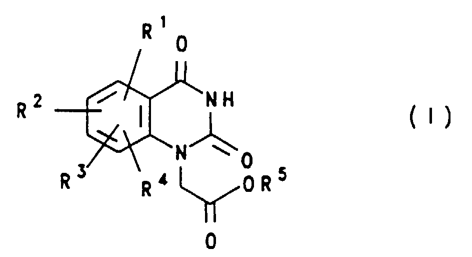 worin
R¹, R², R³, R⁴ unabhängig voneinander Wasserstoff, Halogen, NO₂, (C₁-C₆)Alkoxy, (C₁-C₆)Alkyl, halogensubstituiertes (C₁-C₆)Alkyl
R⁵ Wasserstoff, (C₁-C₆)Alkyl, Phenyl, wobei der Alkyl- oder Phenylrest auch mit Halogenatomen substituiert sein kann, bedeuten,
dadurch gekennzeichnet, daß man ein Anthranilsäurederivat der Formel (II) 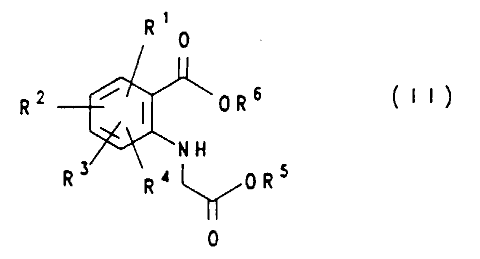 worin
R¹ bis R⁵ die oben erwähnte Bedeutung besitzen und R⁶ für Wasserstoff, (C₁-C₆)Alkyl oder Phenyl steht, wobei der Alkyl- oder Phenylrest auch mit Halogenatomen substituiert sein kann,
mit einem Metallcyanat und Chlorwasserstoff in Gegenwart eines inerten Lösungsmittels umsetzt.

2. Verfahren nach Anspruch 1, dadurch gekennzeichnet, daß R¹, R², R³, R⁴ unabhängig voneinander Wasserstoff, Fluor, Chlor, (C₁-C₄)Alkoxy, (C₁-C₄)Alkyl, chlor- oder fluorsubstituierte (C₁-C₄)Alkyl und
R⁵, R⁶ Wasserstoff, (C₁-C₄)Alkyl, Phenyl bedeuten.

3. Verfahren nach Anspruch 1, dadurch gekennzeichnet, daß
R¹, R², R³, R⁴ unabhängig voneinander Wasserstoff, Fluor, Chlor, Methyl oder Ethyl
und
R⁵, R⁶ Wasserstoff, Methyl oder Ethyl
bedeuten.

4. Verfahren nach mindestens einem der Ansprüche 1 bis 3, dadurch gekennzeichnet, daß zwei, insbesondere drei der Reste R¹, R², R³, R⁴ für Wasserstoff stehen.

5. Verfahren nach Anspruch 1, dadurch gekennzeichnet, daß Formel (I) für 1,2,3,4-Tetrahydro-7-chloro-2,4-dioxochinazolin-1-yl-essigsäureethylester 1,2,3,4-Tetrahydro-7-chloro-2,4-dioxochinazolin-1-ylessigsäuremethylester oder 1,2,3,4-Tetrahydro-7-chloro-2,4-dioxochinazolin-1-ylessigsäure steht.

6. Verfahren nach mindestens einem der Ansprüche 1 bis 5, dadurch gekennzeichnet, daß als inertes Lösungsmittel ein polar aprotisches Lösungsmittel, insbesondere Sulfolan, Dimethylsulfoxid, Dimethylsulfon, Diphenylsulfon oder Tetramethylharnstoff, bevorzugt Sulfolan eingesetzt wird.

7. Verfahren nach mindestens einem der Ansprüche 1 bis 6, dadurch gekennzeichnet, daß als Metallcyanat ein Alkali- oder Erdalkalicyanat, insbesondere Natrium- oder Kaliumcyanat eingesetzt wird.

8. Verfahren nach mindestens einem der Ansprüche 1 bis 7, dadurch gekennzeichnet, daß die Konzentration der Anthranilsäure in dem Lösungsmittel 1 bis 50 Gew.-%, insbesondere bei 1.5 bis 20 Gew.-%, bevorzugt 3 bis 10 Gew.-% beträgt.

9. Verfahren nach mindestens einem der Ansprüche 1 bis 8, dadurch gekennzeichnet, daß die Metallcyanate in Mengen von 0.8 bis 20 Mol Äquivalenten, insbesondere 2 bis 5 Mol Äquivalenten, bezogen auf die Anthranilsäurederivate, zugesetzt werden.

10. Verfahren nach mindestens einem der Ansprüche 1 bis 9, dadurch gekennzeichnet, daß der Chlorwasserstoff gasförmig oder als nichtwäßrige Lösung zugegeben wird.

11. Verfahren nach mindestens einem der Ansprüche 1 bis 10, dadurch gekennzeichnet, daß das Anthranilsäurederivat und das Metallcyanat im Lösungsmittel vorgelegt und Chlorwasserstoff eindosiert wird.

12. Verfahren nach mindestens einem der Ansprüche 1 bis 10, dadurch gekennzeichnet, daß das Anthranilsäurederivat und Chlorwasserstoff vorgelegt wird und das Metallcyanat eindosiert wird.

13. Verfahren nach mindestens einem der Ansprüche 1 bis 10, dadurch gekennzeichnet, daß Chlorwasserstoff und das Metallcyanat vorgelegt und das Anthranilsäurederivat eindosiert wird.

14. Verfahren nach mindestens einem der Ansprüche 1 bis 13, dadurch gekennzeichnet, daß die Reaktionstemperatur zwischen 0° und 100°C, insbesondere zwischen 20 und 75°C liegt.

15. Verfahren nach mindestens einem der Ansprüche 1 bis 14, dadurch gekennzeichnet, daß die Reaktionszeit zwischen 15 min und 24 h, insbesondere zwischen 1 und 15 h, bevorzugt zwischen 2 und 10 h beträgt.

## Claims

1. A process for the preparation of a 1,2,3,4-tetrahydro-2,4-dioxo-quinazolin-l-ylacetic acid derivative of the formula (I) 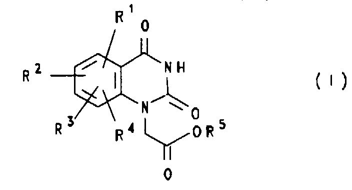 in which
R¹, R², R³, R⁴ independently of one another are hydrogen, halogen, NO₂, (C₁-C₆)alkoxy, (C₁-C₆)alkyl or halogen-substituted (C₁-C₆)alkyl and
R⁵ is hydrogen, (C₁-C₆)alkyl or phenyl, where the alkyl or phenyl radical can also be substituted by halogen atoms,
which comprises reacting an anthranilic acid derivative of the formula (II) 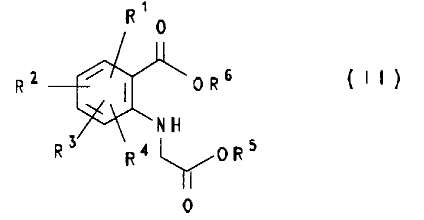 in which
R¹ to R⁵ have the abovementioned meaning and R⁶ is hydrogen, (C₁-C₆)alkyl or phenyl, where the alkyl or phenyl radical can also be substituted by halogen atoms, with a metal cyanate and hydrogen chloride in the presence of an inert solvent.

2. The process as claimed in claim 1, wherein R¹, R², R³ and R⁴ independently of one another are hydrogen, fluorine, chlorine, (C₁-C₄)alkoxy, (C₁-C₄)alkyl or chlorine- or fluorine-substituted (C₁-C₄)alkyl
and
R⁵ and R⁶ are hydrogen, (C₁-C₄)alkyl or phenyl.

3. The process as claimed in claim 1, wherein
R¹, R², R³ and R⁴ independently of one another are hydrogen, fluorine, chlorine, methyl or ethyl and
R⁵ and R⁶ are hydrogen, methyl or ethyl.

4. The process as claimed in at least one of claims 1 to 3, wherein two, and in particular three, of the radicals R¹, R², R³ and R⁴ are hydrogen.

5. The process as claimed in claim 1, wherein formula (I) is ethyl 1,2,3,4-tetrahydro-7-chloro-2,4-dioxoquinazolin-1-ylacetate, methyl 1,2,3,4-tetrahydro-7-chloro-2,4-dioxoquinazolin-1-ylacetate or 1,2,3,4-tetrahydro-7-chloro-2,4-dioxoquinazolin-1-ylacetic acid.

6. The process as claimed in at least one of claims 1 to 5, wherein a polar aprotic solvent, in particular sulfolane, dimethyl sulfoxide, dimethyl sulfone, diphenyl sulfone or tetramethylurea, preferably sulfolane, is employed as the inert solvent.

7. The process as claimed in at least one of claims 1 to 6, wherein an alkali metal cyanate or alkaline earth metal cyanate, in particular sodium cyanate or potassium cyanate, is employed as the metal cyanate.

8. The process as claimed in at least one of claims 1 to 7, wherein the concentration of the anthranilic acid in the solvent is 1 to 50% by weight, in particular 1.5 to 20% by weight, preferably 3 to 10% by weight.

9. The process as claimed in at least one of claims 1 to 8, wherein the metal cyanate is added in an amount of 0.8 to 20 molar equivalents, in particular 2 to 5 molar equivalents, based on the anthranilic acid derivative.

10. The process as claimed in at least one of claims 1 to 9, wherein the hydrogen chloride is added in gaseous form or as a non-aqueous solution.

11. The process as claimed in at least one of claims 1 to 10, wherein the anthranilic acid derivative and the metal cyanate are initially introduced into the solvent and hydrogen chloride is metered in.

12. The process as claimed in at least one of claims 1 to 10, wherein the anthranilic acid derivative and hydrogen chloride are initially introduced and the metal cyanate is metered in.

13. The process as claimed in at least one of claims 1 to 10, wherein hydrogen chloride and the metal cyanate are initially introduced and the anthranilic acid derivative is metered in.

14. The process as claimed in at least one of claims 1 to 13, wherein the reaction temperature is between 0 and 100°C, in particular between 20 and 75°C.

15. The process as claimed in at least one of claims 1 to 14, wherein the reaction time is between 15 minutes and 24 hours, in particular between 1 and 15 hours, preferably between 2 and 10 hours.

## Revendications

1. Procédé de préparation de dérivés 1,2,3,4-tétrahydro-2,4-dioxoquinazolin-l-yl-acétique de formule (I) 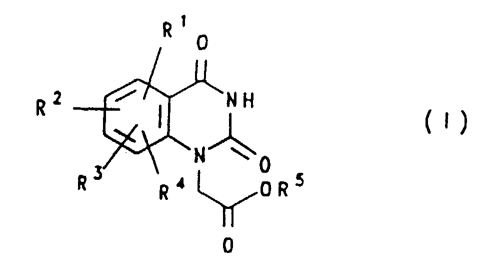 dans laquelle
R¹, R², R³, R⁴ représentent indépendamment les uns des autres un atome d'hydrogène, d'halogène, un groupe NO₂, alcoxy en C₁-C₆, alkyle en C₁-C₆, alkyle en C₁-C₆ substitué par des halogènes,
R⁵ représente un atome d'hydrogène, un groupe alkyle en C₁-C₆, un groupe phényle, dans lequel le groupe alkyle ou phényle peut également être substitué par des halogènes,
caractérisé en ce qu'on met à réagir un dérivé d'acide anthranilique de formule (II) 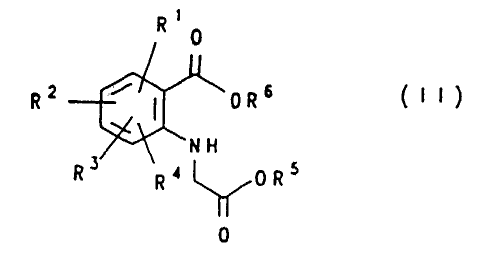 dans laquelle
R¹ à R⁵possèdent la signification ci-dessus mentionnée et R⁶ représente un atome d'hydrogène, un groupe alkyle en C₁-C₆ ou phényle, le groupe alkyle ou phényle pouvant également être substitué par des atomes d'halogène,
avec un cyanate de métal et le chlorure d'hydrogène en présence d'un solvant inerte.

2. Procédé selon la revendication 1, caractérisé en ce que R¹, R², R³, R⁴ représentent indépendamment les uns des autres un atome d'hydrogène, de fluor, de chlore, un groupe alcoxy en C₁-C₄, alkyle en C₁-C₄, alkyle en C₁-C₄ substitué par des atomes de chlore ou de fluor
et
R⁵, R⁶ représentent un atome d'hydrogène, un groupe alkyle en C₁-C₄, phényle.

3. Procédé selon la revendication 1, caractérisé en ce que
R¹, R², R³, R⁴ représentent indépendamment les uns des autres un atome d'hydrogène, de fluor, de chlore, un groupe méthyle ou éthyle
et R⁵, R⁶ représentent un atome d'hydrogène, un groupe méthyle ou éthyle.

4. Procédé selon au moins une des revendications 1 à 3, caractérisé en ce que deux, de préférence trois des groupes R¹, R², R³, R⁴ représentent un atome d'hydrogène.

5. Procédé selon la revendication 1, caractérisé en ce que la formule (I) représente le 1,2,3,4-tétrahydro-7-chloro-2,4-dioxoquinazolin-l-ylacétate d'éthyle, le 1,2,3,4-tétrahydro-7-chloro-2,4-dioxoquinazolin-1-ylacétate de méthyle ou l'acide 1,2,3,4-tétrahydro-7-chloro-2,4-dioxoquinazolin-l-ylacétique.

6. Procédé selon au moins une des revendications 1 à 5, caractérisé en ce qu'on utilise comme solvant inerte un solvant organique aprotique polaire, en particulier le sulfolane, le diméthylsulfoxyde, la diméthylsulfone, la diphénylsulfone ou la tétraméthylurée, de préférence le sulfolane.

7. Procédé selon au moins une des revendications 1 à 6, caractérisé en ce qu'on utilise comme cyanate de métal un cyanate de métal alcalin ou alcalino-terreux, en particulier le cyanate de sodium ou de potassium.

8. Procédé selon au moins une des revendications 1 à 7, caractérisé en ce que la concentration de l'acide anthranilique dans le solvant se situe entre 1 et 50% en poids, en particulier entre 1,5 et 20% en poids, de préférence entre 3 et 10% en poids.

9. Procédé selon au moins une des revendications 1 à 8, caractérisé en ce qu'on ajoute les cyanates de métaux en quantités de 0,8 à 20 équivalents molaires, en particulier de 2 à 5 équivalents molaires, par rapport aux dérivés de l'acide anthranilique.

10. Procédé selon au moins une des revendications 1 à 9, caractérisé en ce qu'on ajoute le chlorure d'hydrogène sous forme gazeuse ou d'une solution non aqueuse.

11. Procédé selon au moins une des revendications 1 à 10, caractérisé en ce qu'on charge l'acide anthranilique et le cyanate de métal dans le solvant et en ce qu'on ajoute ensuite de manière dosée le chlorure d'hydrogène.

12. Procédé selon au moins une des revendications 1 à 10, caractérisé en ce qu'on charge le chlorure d'hydrogène et l'acide anthranilique et en ce qu'on ajoute de manière dosée le cyanate de métal.

13. Procédé selon au moins une des revendications 1 à 10, caractérisé en ce qu'on charge le chlorure d'hydrogène et le cyanate de métal et en ce qu'on ajoute de manière dosée le dérivé de l'acide anthranilique.

14. Procédé selon au moins une des revendications 1 à 13, caractérisé en ce que la température de réaction se situe entre 0 et 100°C, en particulier entre 20 et 75°C.

15. Procédé selon au moins une des revendications 1 à 14, caractérisé en ce que le temps de réaction est entre 15 min. et 24 h, en particulier entre 1 et 15 h, de préférence entre 2 et 10 h.
